# DEMANDE DE BREVET EUROPEEN

(11) **EP 1 897 526 A1**
(43) Date de publication de la demande: **12.03.2008**
(21) Numéro de dépôt: 07113248.4
(22) Date de dépôt: 26.07.2007
(51) Int. Cl.: A61K 8/04, A61K 8/81, A61K 8/89, A61Q 1/00, A61Q 1/02, A61Q 1/04, A61Q 1/08, A61Q 1/10, A61Q 19/04, A61Q 19/00, A61Q 19/08, A61Q 17/04, A61K 8/892

(54) **Dispersion cosmétique huile-dans-eau**

(30) Priorité: 25.08.2006 FR 0653472
(71) Demandeur: L'Oréal, 75008 Paris (FR)
(72) Inventeur: Lemoine, Cyril, 78210, Saint Cyr l'Ecole (FR); Monello, Aldo, 91600, Savigny sur Orge (FR)
(74) Mandataire: Kromer, Christophe

(57) **Abrégé**

L'invention concerne une composition sous forme de dispersion huile-dans-eau comprenant :
- au moins 60 % en poids d'eau, par rapport au poids total de la composition,
- un polymère d'acrylate de glycéryle,
- un homopolymère d'un monomère à groupement sulfonique,
- une huile volatile en une teneur inférieure ou égale à 20 % en poids, par rapport au poids total de la composition,
- et de 0 à 0,5 % d'huile non volatile.

Application au soin de la peau.

## Description

La présente invention concerne une composition cosmétique sous forme d'une dispersion huile-dans-eau comprenant un mélange de polymères ainsi qu'un procédé de traitement cosmétique des matières kératiniques.

Les compositions cosmétiques de soin de la peau se présentent souvent sous forme d'émulsions eau-dans-huile, huile-dans-eau ou des émulsions multiples eau-dans-huile-dans-eau pour obtenir un effet sensoriel sur la peau agréable. En effet, l'application d'une solution aqueuse sur la peau n'est pas agréable pour l'utilisatrice et à tendance à assécher la peau à la différences des huiles comme l'huile d'amande d'abricot qui nourrissent la peau et évitent son assèchement. De plus, une solution aqueuse appliquée sur la peau ne s'étale pas correctement si elle est trop fluide et ne permet donc pas d'obtenir un dépôt homogène sur la peau.

D'autre part, les émulsions ont des inconvénients. Certains tensioactifs nécessaires à la stabilité de l'émulsion comme les savons d'acides gras, par exemple les savons d'acide stéarique, sont inconfortables pour la peau car ils provoquent des irritations et sont également desséchants.
En outre, lorsque les émulsions contiennent des actifs hydrophiles, la présence la présence de corps gras non volatiles comme les huiles non volatiles (huiles végétales, huile d'origine minérale, les huiles siliconées non volatiles) ou les cires forment un film résiduel sur la peau qui nuit à une bonne pénétration de l'actif qui est retenu à la surface de la peau. Par ailleurs, la présence de tensioactif émulsionnant peut perturber la bonne efficacité de l'actif hydrophile, ce dernier pouvant être moins disponible pour bien pénétrer dans la peau.

La présence de polymère épaississant permet de réduire voire supprimer la présence de tensioactifs émulsionnants dans les émulsions. Mais certains épaississants présence aussi des inconvénients. L'hydroxyéthylcellulose forme un film cassant, poudré et en présence de certains actifs comme les sels et les vitamines la viscosité de la composition est fortement abaissée. Les gommes de xanthane ou de guar présentent un toucher gluant, ou confèrent un aspect filant à la composition ; la composition épaissie est longue à pénétrer lors de son application sur la peau. La polyvinylpyrrolidone à tendance à former un film trop rigide et donc cassant. il est donc ainsi difficile de trouver le bon compromis entre une bonne texture épaissie de la composition et une bonne application sur les matières kératiniques, notamment sur la peau, pour permettre notamment une bonne pénétration de la composition et des actifs dans la peau.
De plus, il est souhaitable lors de l'étalement de la composition sur la peau, la texture de la composition reste constante lors de l'application sans présenter de brusque variation qui engendre une sensation de frein désagréable pour l'utilisateur.

Le but de la présente invention est donc de disposer d'une composition cosmétique dont l'application sur les matières kératiniques, notamment sur la peau soit confortable, douce, sans sensation de frein lors de son étalement, ni d'effet collant.

Un autre but de l'invention est de disposer d'une composition de soin de la peau permettant une bonne pénétration des actifs cosmétiques ou dermatologiques hydrophiles dans la peau et donc présentant une bonne efficacité cosmétique (par exemple une bonne efficacité anti-rides).

Les inventeurs ont découvert qu'une telle composition peut être obtenue en formulant une dispersion huile-dans-eau en présence d'un mélange d'épaississants particuliers.

De façon plus précise, l'invention concerne une composition sous forme de dispersion huile-dans-eau comprenant :
- au moins 70 % en poids d'eau, par rapport au poids total de la composition,
- un polymère d'acrylate de glycéryle,
- un homopolymère d'un monomère à groupement sulfonique,
- une huile volatile en une teneur inférieure ou égale à 20 % en poids, par rapport au poids total de la composition,
- et de 0 à 0,5 % d'huile non volatile.

La composition selon l'invention après application sur la peau présente de bonnes propriétés cosmétiques de douceur, de confort. De plus, lorsque la composition contient un actif hydropohile, elle contribue à une bonne pénétration de l'actif favorisant une bonne activité cosmétique (par exemple une bonne efficacité antirides).

L'invention a également pour objet un procédé non thérapeutique de soin ou de maquillage des matières kératiniques comprenant l'application sur les matières kératiniques de la composition définie précédemment.

La composition selon l'invention comprend un polymère d'acrylate de glycéryle qui est un épaississant hydrophile.

De préférence, le polymère d'acrylate de glycéryle est choisi parmi les copolymères d'acrylate de glycéryle et d'acide acrylique. De tels copolymères sont notamment vendus sous les dénominations « LUBRAJEL^{®} MS », « LUBRAJEL^{®} CG », « LUBRAJEL^{®} DV », « LUBRAJEL^{®} NP », « LUBRAJE^{®}L Oll », « LUBRAJEL^{®} Oil BG », « LUBRAJEL^{®} PF », « LUBRAJEL^{®} TW », « LUBRAJEL^{®} WA » par la société Guardian Laboratories. On utilise de préférence le « LUBRAJEL^{®} MS ».

Le polymère d'acrylate de glycéryle peut être présent dans la composition selon l'invention en une teneur allant de 0,01 % à 5 % en poids, par rapport au poids total de la composition, de préférence allant de 0,05 % à 3 % en poids, et préférentiellement allant de 0,1 à 3 % en poids.

Avantageusement, la composition selon l'invention comprend un polymère comportant au moins un monomère à groupement sulfonique. La présence de ce polymère permet d'obtenir une composition présentant de bonnes propriétés cosmétiques, en particulier de non filant notamment lors de la prise au doigt de la composition, de non collant, de douceur, et favorise également une bonne pénétration de la composition lors de son application sur la peau.

Les polymères comportant au moins un monomère à groupement sulfonique, utilisés dans la composition de l'invention, sont hydrosolubles ou hydrodispersibles ou gonflables dans l'eau. Les polymères utilisés conformément à l'invention sont des homopolymères susceptibles d'être obtenus à partir d'au moins un monomère à insaturation éthylénique et à groupement sulfonique, pouvant être sous forme libre ou partiellement ou totalement neutralisée.

De façon préférentielle, les polymères conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non réticulés.

Les monomères à groupement sulfonique du polymère utilisé dans la composition de l'invention sont choisis notamment parmi l'acide vinylsulfonique, l'acide styrènesulfonique, les acides (méth)acrylamido-(C₁-C₂₂)alkylsulfoniques, les acides N-(C₁-C₂₂)alkyl-(méth)acrylamido-(C₁-C₂₂)-alkylsulfoniques comme l'acide undécyl-acrylamido-méthane-sulfonique, ainsi que leurs formes partiellement ou totalement neutralisées, et leurs mélanges.

Selon un mode préféré de réalisation de l'invention, les monomères à groupement sulfonique sont choisis parmi les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide 2-méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées, et leurs mélanges.

Plus particulièrement, on utilise l'acide 2-acrylamido-2-méthylpropane-sulfonique (AMPS) ainsi que ses formes partiellement ou totalement neutralisées.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

L'homopolymère de monomères à groupement sulfonique peut être réticulé avec un ou plusieurs agents de réticulation.

Ces homopolymères sont généralement réticulés et neutralisés, et ils peuvent être obtenus selon le procédé de préparation comprenant les étapes suivantes :
(a) on disperse ou on dissout le monomère tel que l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les homopolymères d'AMPS préférés sont en général caractérisés par le fait qu'ils comprennent, distribués de façon aléatoire :
a) de 90 à 99,9% en poids de motifs de formule générale (II) suivante : 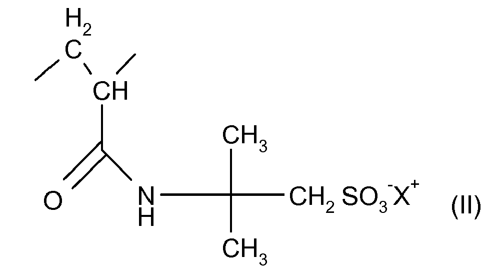 dans laquelle X⁺ désigne un proton, un cation de métal alcalin, un cation alcalino-terreux ou l'ion ammonium, au plus 10% mol des cations X⁺ pouvant être des protons H⁺ ;
b) de 0,01 à 10% en poids de motifs réticulants provenant d'au moins un monomère ayant au moins deux double-liaison oléfiniques ; les proportions en poids étant définis par rapport au poids total du polymère.

Les homopolymères selon l'invention plus particulièrement préférés comprennent de 98 à 99,5 % en poids de motifs de formule (II) et de 0,2 à 2 % en poids de motifs réticulants.

Comme polymères de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS » (nom CTFA: ammonium polyacryldimethyltauramide).

L'homopolymère de monomère à groupement sulfonique peut être présent dans la composition selon l'invention en une teneur en matière active allant par exemple de 0,01 à 10 % en poids, de préférence allant de 0,1 à 5 % en poids, préférentiellement allant de 0,1 à 3 % en poids, par rapport au poids total de la composition.

Avantageusement, le polymère d'acrylate de glycéryle et l'homopolymère de monomère à groupement sulfonique décrits précédemment peuvent être présents dans la composition selon l'invention en un ratio pondéral polyacrylate de glycéryle/homopolymère de monomère à groupement sulfonique allant de 1 à 4, de préférence allant de 1,5 à 3, préférentiellement allant de 1,5 à 2,5, et plus préférentiellement allant de 1,8 à 2,3.

La composition selon l'invention peut comprendre jusqu'à 20 % en poids d'huile volatile, par rapport au poids total de la composition, notamment en une teneur allant de 0,1 % à 20 % en poids, par rapport au poids total de la composition, de préférence allant de 0,5 % à 15 % en poids, préférentiellement allant de 1 % à 10 % en poids, et plus préférentiellement allant de 5 % à 10 % en poids. L'huile volatile permet une étalement facile et agréable de la composition sur la peau.

L'huile volatile peut être choisie parmi les huiles volatiles siliconées, les huiles volatiles non siliconées.

Par huile volatile, on entend tout milieu non aqueux susceptible de s'évaporer de la peau ou des lèvres, en moins d'une heure, ayant notamment une pression de vapeur, à température ambiante et pression atmosphérique allant de 10⁻³ à 300 mm de Hg (0,13 Pa à 40 000 Pa).

Comme huile volatile utilisable dans l'invention, on peut utiliser les huiles volatiles non siliconées, notamment les isoparaffines en C₈-C₁₆ comme l'isododécane, l'isodécane, l'iso-hexadécane et par exemple les huiles vendues sous les noms commerciaux d'ISOPAR, de PERMETHYL et notamment l'isododécane (PERMETHYL 99 A).

Comme huile siliconée volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Avantageusement, la composition selon l'invention peut comprendre jusqu'à 0,5 % (notamment de 0,1 à 0,5 %) en poids, par rapport au poids total de la composition, de tensioactif ayant un HLB supérieure ou égal à 14, notamment allant de 14 à 18 , et de préférence allant de 14 à 16. Un tel tensioactif ne contribue pas à émulsionner la phase huileuse dans la phase aqueuse mais permet de solubiliser un ou des parfums qui peuvent être présents dans la composition.

Avantageusement, la composition selon l'invention ne comprend pas de tensioactif émulsionnant ayant un HLB (hydrophile-lipophile-balance) inférieur à 14, c'est-à-dire qu'un tel émulsionnant est présent en une teneur inférieure à 0,5 % en poids, par rapport au poids total de la composition, voire est exempt (teneur nulle) de la composition.

La valeur HLB selon GRIFFIN est définie dans J. Soc. Cosm. Chem. 1954 (volume 5), pages 249-256. On peut se reporter au document "Encyclopedia of Chemical Technology, KIRK-OTHMER", volume 22, p. 333-432, 3ème édition, 1979, WILEY, pour la définition des propriétés et des fonctions (émulsionnant) des tensioactifs, en particulier p. 347-377 de cette référence, pour les tensioactifs non ioniques.

Comme exemple de tensioactif ayant un HLB supérieur à 14, on peut citer les esters d'acides gars et de polyéthylèneglycol comme l'huile de ricin hydogénée éthoxylée , comportant notamment de 30 à 70 motifs d'oxyde d'éthylène, tels que ceux vendus sous les dénominations Cremophor^{®} RH40 et Cremophor^{®} RH 60 par la société BASF. On peut citer également le nonylphénol oxyéthyléné, notamment à 12 motifs d'oxyde d'éthylène, comme celui vendu sous la dénomination « Solubilisant S 12 » par la société Givaudan.

La composition selon l'invention contient moins de 0,5 % (ou de 0 à 0,5 % en poids) en poids d'huile non volatile, par rapport au poids total de la composition, voire est exempte (teneur nulle) d'huile non volatile.

On entend par huile non volatile une huile susceptible de rester sur la peau à température ambiante (25 °C) et pression atmosphérique au moins une heure et ayant notamment une pression de vapeur à température ambiante (25 °C) et pression atmosphérique, non nulle, inférieure à 0,01 mm de Hg (1,33 Pa).

Comme huile non volatile utilisable dans l'invention, on peut citer :
- les huiles non volatile non siliconées, notamment hydrocarbonées, telles que l'huile de paraffine (ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C₁₂-C₃₆, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine ; les acides gras supérieurs, notamment en C₁₄-C₂₂, tels que l'acide myristique, l'acide palmitique, l'acide stéarique, l'acide béhénique, l'acide oléique, l'acide linoléique, l'acide linolénique ou l'acide isostéarique ; les alcools gras supérieurs, notamment en C₁₆- C₂₂, tels que le cétanol, l'alcool oléique, l'alcool linoléique ou linolénique, l'alcool isostéarique ou l'octyl dodécanol ; et leurs mélanges.
- les huiles siliconées non volatiles telles que les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes ; les polysiloxanes modifiés par des acides gras (notamment en C₈-C₂₀), des alcools gras (notamment en C₈-C₂₀) ou des polyoxyalkylènes (notamment polyoxyéthyléne et/ou polyoxypropylène) ; les silicones aminées ; les silicones à groupement hydroxyles ; les silicones fluorés comportant un groupement fluoré pendant ou en bout de chaîne siliconée ayant de 1 à 12 atomes de carbone dont tout ou partie des hydrogène sont substitués par des atomes de fluor ; et leurs mélanges

La composition selon l'invention comprend une phase aqueuse.

La composition peut comprendre de l'eau en une teneur allant de 60% à 95 % en poids, par rapport au poids total de la composition, de préférence allant de 65 % à 90 % en poids, et préférentiellement allant de 70 % à 80 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre en outre un polyol miscible à l'eau à la température ambiante (25 °C) notamment choisi parmi les polyols ayant notamment de 2 à 20 atomes de carbones, de préférence ayant de 2 à 10 atomes de carbone, et préférentiellement ayant de 2 à 6 atomes de carbones, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ; et leurs mélanges.

La composition selon l'invention peut comprendre un polyol miscible à l'eau à la température ambiante en une teneur allant de 1 % à 20 % en poids, par rapport au poids total de la composition, et de préférence allant de 3 % à 15 % en poids.

En outre, la composition selon l'invention peut comprendre un monoalcool ayant de 2 à 6 atomes de carbone tel que l'éthanol, l'isopropanol en une teneur inférieure ou égale à 5 % en poids, par rapport au poids total de l'invention, notamment allant de 0,01 à 5 % en poids. Selon un mode de réalisation particulier de l'invention, la composition peut être exempte d'un tel monoalcool (teneur nulle).

La composition peut comprendre des charges en une teneur inférieure ou égale à 3 % en poids, par rapport au poids total de la composition, notamment en une teneur allant de 0,1 à 3 % en poids. Selon un mode de réalisation selon l'invention, la composition est exempte de charges (teneur nulle).

Par "charge", on entend toute particule incolore choisie parmi les charges minérales ou organiques, lamellaires, sphériques ou oblongues, chimiquement inerte dans la composition.

On peut citer le talc, le mica, la silice, le kaolin, la laponite, les poudres de polyamide comme le Nylon®, les poudres de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile telles que l'Expancel® (Nobel Industrie), les particules de polymère acrylique, notamment de copolymère d'acide acrylique comme le Polytrap® (Dow Corning), les poudres de polyuréthane, les microbilles de résine de silicone (Tospearls® de Toshiba, par exemple), le carbonate de calcium précipité, le dicalcium phosphate, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

La composition comprend avantageusement un actif cosmétique ou dermatologique hydrophile. L'actif hydrophile peut être n'importe quelle molécule hydrosoluble ayant une activité cosmétique ou dermatologique et ayant une solubilité dans l'eau d'au moins 0,25 % en poids à température ambiante (25°C).

Les actifs hydrophiles peuvent être notamment choisis parmi les actifs dermorelaxants, les agents stimulant la synthèse des fibres de la matrice extra-cellulaire dermique ou épidermique ou des fibres localisées au niveau de la jonction dermo-épidermique et/ou empêchant leur dégradation, les agents anti-glycation, les actifs lipolytiques ou inhibant la lipogénèse, les agents anti-irritants, et leurs mélanges. De tels actifs hydrophiles sont décrits par exemples dans la demande FR-A-2877221.

On peut également citer à titre d'exemple :
- les agents anti-radicaux libres et/ou détoxifiants tels que l'acide ascorbique et les dérivés de celui-ci comme l'ascorbylphosphate de magnésium, les dérivés de la cystéine comme par exemple la N-acétylcystéine, les protéines et les enzymes, par exemple la superoxyde dismutase (SOD), les peroxydases telles que la lactoperoxydase et la lactoferrine, la catalase, les protéases telles que la subtillisine et la papaïne, les lipases, l'uricase, les peptides et leurs dérivés, l'ubiquinone et le cytochrome C,
- les agents kératolytiques tels que les alpha-hydroxyacides, les bêta-hydroxyacides et les alpha-cétoacides comme l'acide salicylique et ses dérivés,
- les accélérateurs de bronzage tels que les dérivés de tyrosine,
- les actifs dépigmentants tels que l'acide kojique, l'arbutine et les dérivés de ceux-ci,
- les actifs autobronzants tels que la dihydroxyacétone et les indoles,
- les liporégulateurs tels que la caféine et la théophylline,
- les agents hydratants tels que le sorbitol, le xylitol, l'urée et l'ADN végétal,
- les agents antipelliculaires tels que la piroctone olamine et les dérivés de pyridine thione,
- les agents antiélastases comme l'acide {2-[acétyl-(3-trifluorométhyl-phényl)-amino]-3-méthyl-butyrylaminol acétique (composé décrit dans la demande WO 01/94381);
- les agents dermorelaxants tels que l'adénosine ;
- et leurs mélanges.

Préférentiellement, l'actif hydrophile est choisi parmi l'acide {2-[acétyl-(3-trifluorométhylphényl)-amino]-3-méthyl-butyrylamino} acétique, l'adénosine, la caféine, et leurs mélanges.

Le ou les actif(s) hydrophile(s) peu(ven)t être présent(s) dans la composition selon l'invention en une teneur allant de 0,001 % à 10 % en poids, de préférence allant de 0,01 % à 5 % en poids, et préférentiellement allant de 0,05 % à 1 % en poids, par rapport au poids total de la composition.

Avantageusement, la composition selon l'invention contient moins de 0,5 % en poids de cire, par rapport au poids total de la composition voire est exempte de cire (teneur nulle).

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.
Le point de fusion de la cire peut être mesuré à l'aide d'un calorimètre à balayage différentiel (D.S.C.), par exemple le calorimètre vendu sous la dénomination DSC 30 par la société METLER.

La composition selon l'invention peut comprendre également les adjuvants habituels dans les domaines cosmétique et dermatologique, tels que les conservateurs, les antioxydants, les parfums, les charges, les pigments, les filtres UV, les absorbeurs d'odeur et les matières colorantes. Les quantités de ces différents adjuvants sont celles classiquement utilisées dans les domaines considérés, et par exemple de 0,01 à 20 % du poids total de la composition.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La composition peut être une composition de soin, notamment être un produit de soin de la peau tels qu'une base de soin pour la peau, une crème de soin (crème de jour, de nuit, anti-rides), une base de maquillage ; une composition de soin pour les lèvres (baume à lèvres) ; une composition de protection solaire ou autobronzante.

La composition peut également être une composition de maquillage, notamment de maquillage de la peau, comme un fond de teint, un fard à joue, un fard à paupière, un produit anti-cernes, un produit de maquillage du corps.

Les exemples qui suivent illustrent l'invention.

### Exemple 1 :

On a préparé une composition de soin du visage antirides contenant les ingrédients suivants :
- Acétyl trifluorométhylphényl valylglycine* 0,08 g
- Caféine 0,5 g
- Mélange de Saccharomyces cerevisiae , de mannitol, de cyclodextrine et disodium succinate (CYTOVITIN LS 9388 de chez Laboratoires Serobiologiques) 0,2 g
- Adénosine 0,04 g
- Hydrolysat de protéine de soja (Phytokine^{®} de chez Coletica) 0,4 g
- Extrait de Lentinus Edodes à 5,5 % dans l'eau (Ferminskin^{®} de chez Silab) 0,4 g
- Acide poly acrylamidométhyl propane sulfonique neutralisé partiellement à l'ammoniaque et hautement réticulé (Hostacerin AMPS de chez Clariant) 1 g
- Copolymère acrylate de glycéryle/acide acrylique dans eau/glycérine à 1 %
   (LUBRAJEL^{®} MS . de chez Guardian Laboratories) 5 g soit 0,05 g MA
- Fucose à 20 % dans l'eau (Fucogel^{®} 1000 PP de chez Solabia) 2 g
- Cyclopentasiloxane 7 g
- Mélange de cyclopentasiloxane et de dimethiconol (Dow Corning 1501 Fluid) 1 g
- Glycérine 7 g
- Propylène glycol 2 g
- Sel disodique de l'acide éthylènediamine tétracétique 0,15 g
- Triéthanolamine 0,05 g
- Conservateurs qs
- Eau qsp 100 g
*= acide {2-[acétyl-(3-trifluorométhyl-phényl)-amino]-3-méthyl-butyrylamino} acétique autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, préparé comme décrit dans le brevet EP 1 292 608 B1.

La composition appliquée sur la visage est confortable, douce, et présente une bonne efficacité antirides.

On a mesuré l'efficacité antirides de cette composition sur un panel de 37 femmes âgées de 40 à 70 ans, tous types de peau, présentant des rides et ridules au niveau de la patte d'oie.
- Utilisation du produit 2 fois par jour pendant 4 semaines.
- Evaluation du nombre, de la surface, de la longueur des rides par des empreintes prises avec le caoutcouc siliconé vendu sous la dénomination SILFLO^{®} par la société Flexico Developments LTD puis analyse d'image sur le principe de la mesure des ombres portées générées par une illumination en lumière rasante de l'empreinte.

Des empreintes prises sur les rides de la patte d'oie après traitement 4 semaines et 12 semaines, comparées à T 0 ont permis d'évaluer les paramètres suivants :

| | **T 4 semaines/ T 0** | **T 12 semainses / T 0** |
|---|---|---|
| Nombre total de rides | - 9% | - 9% |
| Surface totale ridée | - 23% | - 13% |
| Longueur de la surface totale ridée | - 16% | - 13% |

Les résultats obtenus montrent que la composition permet de réduire le nombre de rides et la surface totale ridée, prouvant ainsi l'efficacité anti-rides de la composition.

On a également évalué les qualité cosmétique de la composition sur un pannel de 51femmes âgées de 40 à 70 ans, tous types de peau, présentant des rides et ridules au niveau de la patte d'oie.
• Utilisation du produit 2 fois par jour pendant 4 semaines.
• Les femmes ont répondu à un questionnaire concernant l'efficacité subjective du produit et les réponses obtenues sont les suivantes :

| | **T immédiat** | **T 4 semaines** |
|---|---|---|
| La peau paraît plus lisse | **92%** | **98%** |
| La peau paraît plus confortable | **92%** | **98%** |
| La peau paraît plus douce | **98%** | **98%** |

Les résultats obtenus montrent que la composition appliquée sur la peau confère un effet plus lisse à la peau ainsi q'un confort et une douceur notables.

## Revendications

1. Composition sous forme de dispersion huile-dans-eau comprenant :
- au moins 60 % en poids d'eau, par rapport au poids total de la composition,
- un polymère d'acrylate de glycéryle,
- un homopolymère d'un monomère à groupement sulfonique,
- une huile volatile en une teneur inférieure ou égale à 20 % en poids, par rapport au poids total de la composition,
- et de 0 à 0,5 % d'huile non volatile.

2. Composition selon la revendication précédente, **caractérisée par le fait que** le polymère d'acrylate de glycéryle est un copolymère d'acrylate de glycéryle et d'acide acrylique.

3. Composition selon l'un quelconque des revendications précédentes, **caractérisée par le fait que** le polymère d'acrylate de glycéryle est présent en une teneur allant de 0,01 % à 5 % en poids, par rapport au poids total de la composition, de préférence allant de 0,05 % à 3 % en poids, et préférentiellement allant de 0,1 à 3 % en poids.

4. Composition selon l'un quelconque des revendications précédentes, **caractérisée par le fait que** le monomère à groupement sulfonique est choisi parmi l'acide vinylsulfonique, l'acide styrènesulfonique, les acides (méth)acrylamido(C₁-C₂₂)alkylsulfoniques, les acides N-(C₁-C₂₂)alkyl-(méth)acrylamido(C₁-C₂₂)alkyl-sulfoniques, ainsi que leurs formes partiellement ou totalement neutralisées, et leurs mélanges.

5. Composition selon l'un quelconque des revendications précédentes, **caractérisée par le fait que** le monomère à groupement sulfonique est l'acide 2-acrylamido 2-méthylpropane sulfonique.

6. Composition selon l'une quelconque des revendications précédentes, **caractérisée par le fait que** l'homopolymère est un homopolymère réticulé et neutralisé d'acide 2-acrylamido 2-méthylpropane sulfonique.

7. Composition selon l'une quelconque des revendications précédentes, **caractérisée par le fait que** l'homopolymère de monomère à groupement sulfonique est présent en une quantité en matière active allant de 0,01 à 10 % en poids, de préférence allant de 0,1 à 5 % en poids, préférentiellement allant de 0,1 à 3 % en poids, par rapport au poids total de la composition.

8. Composition selon l'une quelconque des revendications précédentes, **caractérisée par le fait que** le polymère d'acrylate de glycéryle et l'homopolymère de monomère à groupement sulfonique sont présents en un ratio pondéral polyacrylate de glycéryle/homopolymère de monomère à groupement sulfonique allant de 1 à 4, de préférence allant de 1,5 à 3, préférentiellement allant de 1,5 à 2,5, et plus préférentiellement allant de 1,8 à 2,3.

9. Composition selon l'une quelconque des revendications précédentes, **caractérisée par le fait que** l'huile volatile est présente en une teneur allant de 0,1 % à 20 % en poids, par rapport au poids total de la composition, de préférence allant de 0,5 % à 15 % en poids, préférentiellement allant de 1 % à 10 % en poids, et plus préférentiellement allant de 5 % à 10 % en poids.

10. Composition selon l'une quelconque des revendications précédentes, **caractérisée par le fait qu'**elle est exempte d'huile non volatile.

11. Composition selon l'une quelconque des revendications précédentes, **caractérisée par le fait qu'**elle comprend de l'eau en une teneur allant de 60% à 95 % en poids, par rapport au poids total de la composition, de préférence allant de 65 % à 90 % en poids, et préférentiellement allant de 70 % à 80 % en poids.

12. Composition selon l'une quelconque des revendications précédentes, **caractérisée par le fait qu'**elle comprend un polyol miscible à l'eau à la température ambiante.

13. Composition selon la revendication précédente, **caractérisée par le fait que** le polyol est choisi parmi la glycérine, le propylène glycol, le butylène glycol, le pentylène glycol, l'hexylène glycol, le dipropylène glycol, le diéthylène glycol.

14. Composition selon l'une des revendications 12 ou 13, **caractérisée par le fait que** le polyol miscible à l'eau à la température ambiante est présent en une teneur allant de 1 % à 20 % en poids, par rapport au poids total de la composition, et de préférence allant de 3 % à 15 % en poids.

15. Composition selon l'une quelconque des revendications précédentes, **caractérisée par le fait qu'**elle comprend un monoalcool ayant de 2 à 6 atomes de carbone en une teneur inférieure ou égale à 5 % en poids, par rapport au poids total de l'invention, notamment allant de 0,01 à 5 % en poids.

16. Composition selon l'une quelconque des revendications 1 à 14, **caractérisée par le fait qu'**elle est exempte de monoalcool ayant de 2 à 6 atomes de carbone.

17. Composition selon l'une quelconque des revendications précédentes, **caractérisée par le fait qu'**elle comprend une ou des charges en une teneur inférieure ou égale à 3 % en poids, par rapport au poids total de la composition, notamment en une teneur allant de 0,1 à 3 % en poids.

18. Composition selon l'une quelconque des revendications 1 à 16, **caractérisée par le fait qu'**elle est exempte de charges.

19. Composition selon l'une quelconque des revendications précédentes, **caractérisée par le fait qu'**elle contient moins de 0,5 % en poids de cire, par rapport au poids total de la composition.

20. Composition selon l'une quelconque des revendications précédentes, **caractérisée par le fait qu'**elle comprend un actif cosmétique ou dermatologique hydrophile.

21. Composition selon l'une quelconque des revendications précédentes, **caractérisée par le fait qu'**elle comprend un adjuvant cosmétique choisis parmi les conservateurs, les antioxydants, les parfums, les charges, les pigments, les filtres UV, les absorbeurs d'odeur et les matières colorantes.

22. Procédé non thérapeutique de soin ou de maquillage des matières kératiniques comprenant l'application sur les matières kératiniques d'une composition selon l'une quelconque des revendications précédentes.
